# EUROPEAN PATENT SPECIFICATION

(11) **EP 3 829 535 B1**
(45) Date of publication and mention of the grant of the patent: **08.11.2023**
(21) Application number: 18830309.3
(22) Date of filing: 10.12.2018
(51) Int. Cl.: A61K 9/00, A61K 47/12, A61K 47/18, A61K 31/00, A61K 31/198, A61P 17/00, A61P 27/16

(54) **COMPOSITIONS FOR THE PREVENTION AND TREATMENT OF CUTANEOUS AFFECTIONS**
ZUSAMMENSETZUNGEN ZUR VORBEUGUNG UND BEHANDLUNG VON HAUTKRANKHEITEN
COMPOSITIONS POUR LA PRÉVENTION ET LE TRAITEMENT D'AFFECTIONS CUTANÉES

(30) Priority: 27.07.2018 IT 201800007596
(43) Date of publication of application: 09.06.2021
(73) Proprietor: I.C.F. S.r.l., 26020 Palazzo Pignano (CR) (IT)
(72) Inventor: FALANGA, Gennaro, 26020 Palazzo Pignano (CR) (IT)
(74) Representative: Villa, Livia
(86) International application number: PCT/IB2018/059823
(87) International publication number: WO 2020/021324

(56) References cited:
- EP-B1- 1 711 158
- GB-A- 2 234 171
- JANE LEA ET AL: "In vitro efficacy of N-acetylcysteine on bacteria associated with chronic suppurative otitis media", JOURNAL OF OTOLARYNGOLOGY - HEAD AND NECK SURGERY, BIOMED CENTRAL LTD, LONDON, UK, vol. 43, no. 1, 7 July 2014 (2014-07-07), page 20, XP021191280, ISSN: 1916-0216, DOI: 10.1186/1916-0216-43-20

## Description

### FIELD OF THE INVENTION

The present invention relates to a pharmaceutical composition for the prevention and treatment of external otitis or otitis media, in particular in the veterinary field. The composition of the invention comprises a synergistic association of N-acetylcysteine and a stabilizing agent, which has been shown to be significantly active against pathogens causing the external otitis, at the same time significantly reducing the disadvantages of N-acetylcysteine.

### BACKGROUND ART

The ear is a very delicate organ that allows us to hear and also regulates the sense of balance. Among the most common diseases associated with the ear are external otitis and otitis media. External otitis is the inflammation of the external auditory canal, i.e. the channel that brings to the eardrum the sounds collected by the auricles. The most common symptoms of external otitis are discomfort, itching or ear pain: in some cases, the ear pain (the exact term is otalgia) is very intense and may increase during chewing (the external ear canal is very close to articulation of the mandible) or when pressing with the fingers or exerting stress on the auricle. Otitis media, on the other hand, is the inflammation of the middle ear, i.e. the tympanic cavity or tympanum box which contains the chain of the ossicles (hammer, anvil, stirrup). Like all the inflammations, the otitis media can be acute, then manifests suddenly and then disappears more or less rapidly without leaving any sign, but it can also become chronic, with alternating phases of minor or greater severity.

The presence of germs in the middle ear is responsible for the true and actual otitis, which can manifest with high fever and throbbing pain. In some cases, the infection present in the middle ear may cause the production of pus which may cause a spontaneous rupture of the eardrum membrane. In this sense, bacterial biofilms play a significant role in the pathogenesis of a variety of otorhinolaryngeal diseases, including otitis media and cholesteatoma.

Biofilms allow the survival of bacterial cells in a hostile environment; the extremely complex structure and the metabolic and physiological heterogeneity that characterize them suggest an analogy between these communities and the tissues of higher organisms. Bacterial biofilms, not easily eradicated with conventional antibiotic therapies, affect a large number of chronic bacterial infections.

For the treatment of bacterial infections of the urinary tract, the synergistic effect between phosphomycin and acetylcysteine was determined in the disintegration of biofilms from E. coli. Acetylcysteine (NAC) has determined, at high concentrations, a good reduction in the mass of biofilms, even up to 55%.

N-Acetylcysteine is N-acetylated cysteine, i.e. an amino acid containing a thiol group, also known as α-acetamido-β-mercaptopropanoic acid. Topical cosmetic compositions containing N-acetylcysteine are known to improve the physical appearance of the skin, in particular wrinkles. In fact, since N-acetylcysteine contains a free thiol group, it acts as an antioxidant. However, N-acetylcysteine is associated with a number of significant disadvantages. It is known that N-acetylcysteine degrades under normal conditions of storage and produces a very unpleasant odor. It is believed that this odor derives from the release of thiolate compounds and hydrogen sulfides resulting from the degradation itself, which are immediately recognizable by the pungent note such as "rotten egg". Therefore, topical compositions containing N-acetylcysteine have little or no commercial use due to its unpleasant odor.

GB 2 234 171 discloses aqueous formulations of N-acetylcysteine and stabilizers comprising EDTA and TRIS.

It is therefore an object of the present invention to provide an alternative solution to the use of antibiotic drugs, which allows to prevent and effectively treat external otitis and otitis media, without triggering resistance phenomena typical of antibiotics, and at the same time is acceptable from the point of view of the pleasantness of use and storage manageability.

### SUMMARY OF THE INVENTION

Said object has been achieved by a pharmaceutical composition comprising N-acetylcysteine and a stabilizing agent, as reported in claim 1.

In another aspect, the present invention concerns said composition for use in the prevention and treatment of cutaneous affections and otitis external and media.

The characteristics and advantages of the present invention will be apparent from the following detailed description and the embodiments provided as illustrative and nonlimiting examples.

### DETAILED DESCRIPTION OF THE INVENTION

Therefore, the invention concerns a pharmaceutical composition comprising N-acetylcysteine and a stabilizing agent, wherein
said stabilizing agent comprises
a buffer compound selected from TRIS (or tris(hydroxymethyl) aminomethane), PIPES (or piperazin-1,4-bis(2-ethanesulfonate acid)), TRIS^{∗}HCl, HEPES (or 4-2-hydroxyethyl-1-piperazinyl-ethanesulfonic acid), monobasic and dibasic sodium phosphate, or citric acid, and
a sequestering compound selected from EGTA (ethyleneglycoltetraacetic acid), EDTA (ethylenediaminetetraacetic acid) or its salified anhydrous or hydrated form, calcium-disodium EDTA or its hydrated form, diammonium EDTA or its hydrated form, dipotassium EDTA or its hydrated form, disodium EDTA or its hydrated or dihydrated form, TEA-EDTA (EDTA mono salt (triethanolamine)), tetrasodium EDTA, tripotassium EDTA, trisodium EDTA, HEDTA (hydroxyethyl-ethylenediaminotriacetic acid), HEDTA-EDTA, and mixtures thereof,
said stabilizing agent is in a weight quantity higher than N-acetylcysteine, and
said composition has a pH of 7-9 in water.

In fact, it has surprisingly been found that the stabilizing agent defined above, even in advantageously reduced amounts, allows N-acetylcysteine to be stabilized, thus significantly reducing its degradation over time and thus avoiding the release of odorous compounds. Indeed, as it will be seen from the following Examples, the composition of the invention, after one month at 60°C, then under storage conditions accelerated by thermal stress, was perfectly odorless, as well as limpid as freshly prepared.

Preferably, in the composition of the invention, stabilizing agent and N-acetylcysteine are in a weight ratio of at least 1.2:1.

More preferably, stabilizing agent and N-acetylcysteine are in a weight ratio of not more than 50: 1.

In preferred embodiments, stabilizing agent and N-acetylcysteine are in a weight ratio of 1.5:1 to 20:1.

The compositions wherein the stabilizing agent and N-acetylcysteine are in a weight ratio of 1.5:1 to 10:1 are particularly preferred.

In some embodiments, in the stabilizing agent, said buffer compound is in a quantity higher than said sequestering compound. Preferably, said buffer compound and said sequestering compound are in a weight ratio of 1.1:1 to 20:1, more preferably 1.5:1 to 10:1.

In other embodiments, the composition of the invention comprises up to 5 wt% of N-acetylcysteine, based on the weight of the composition.

Preferably, the composition of the invention comprises up to 3 wt% of N-acetylcysteine, based on the weight of the composition.

More preferably, the composition of the invention comprises 0.1-2 wt% of N-acetylcysteine, based on the weight of the composition.

In further embodiments, the composition of the invention comprises up to 10 wt% of stabilizing agent, based on the weight of the composition.

Preferably, the composition of the invention comprises up to 8 wt% of stabilizing agent, based on the weight of the composition.

More preferably, the composition of the invention comprises 0.1-5 wt% of stabilizing agent, based on the weight of the composition.

Among the buffer compounds, TRIS (or tris (hydroxymethyl) aminomethane) is preferred.

Among the sequestering compounds, EDTA disodium or its hydrated or dihydrate form is preferred.

The composition of the invention can further comprise pharmaceutically acceptable excipients. The term "excipient" refers to a compound or mixture thereof suitable for use in a pharmaceutical composition. For example, an excipient for use in a pharmaceutical formulation generally should not cause an adverse response in a subject, nor should it significantly inhibit the efficacy of the composition.

Suitable excipients can be rheological additives, pH regulators, antioxidant agents, anti-isothermal agents, antistatic agents, absorbent agents, UV absorbing agents, astringent agents, skin conditioning agents, preservative agents, covering agents, denaturing agents, depigmenting agents, emulsifying agents, filmogenic agents, gelling agents, moisturizing agents, hydrotropic agents, soothing agents, smoothing agents, opacifying agents, plasticizing agents, propellants, skin protecting agents, reducing agents, cooling agents, sebum-restoring agents, solvents, emulsifying stabilizing agents, toning agents, agents humectants, and their mixtures.

Among the solvents, water is particularly preferred.

Compositions comprising N-acetylcysteine, TRIS, disodium EDTA, and water are particularly preferred.

As said, the composition of the invention has a pH of 7-9 in water. Preferred excipients therefore comprise pH regulators.

The addition of excipients can be carried out by methods known in the art. In fact, the components can, for example, be mixed as such or with one or more excipients.

The composition of the invention may be in the form of solution, emulsion, suspension, gel, ampoules, drops or sprays.

In some embodiments, the composition of the invention consists essentially of N-acetylcysteine, stabilizing agent, and water. The expression " consists essentially of" means that N-acetylcysteine is the only active ingredient for the prevention and treatment of otitis present in the composition of the invention, while any additional components or excipients do not interfere with this active ingredient, and are miscible and soluble in water.

In other embodiments, the composition of the invention consists of N-acetylcysteine, stabilizing agent, water and pharmaceutically acceptable excipients.

In further embodiments, the composition of the invention consists of N-acetylcysteine, stabilizing agent, and water.

It should be understood that all the aspects identified above as preferred and advantageous for the composition and its components are to be considered similarly preferred and advantageous also for these embodiments.

In another aspect, the present invention relates to the above described pharmaceutical composition for use in the prevention and treatment of cutaneous affections and external otitis and otitis media.

The term "cutaneous affections" means blotches, papules, blisters, pimples, pustules, cysts, erosions, abrasions, redness, ulcers, cracks, sores, telangectasia, desquamation, rashes, crusts, lichenifications, abrasions, hardening, cuts, lacerations, or atrophy. Such cutaneous affections are the manifestation of skin diseases such as dermatitis, digital and interdigital dermatitis, pododermatitis, pyoderma, dermatosis, furunculosis, candidiasis, erythroderma, erythema, burns, folliculitis, trichomycosis, keratitis, eczema, psoriasis, porocheratosis, urticaria, demodectic mange, malasseziasi, parasitic, abscesses, phlegmons, zoppina.

In fact, as said, since the composition thus obtained has been stable over time and therefore odourless, it can be effectively used without the risk of developing unpleasant odours.

The composition of the invention is preferably administered by external topical route. Preferably, said external otitis and otitis media are in animal beings, such as pets. In particular, such pets are dogs and cats.

It should be understood that all the possible combinations of the preferred aspects of the components of the composition, as indicated above, are herein described and therefore similarly preferred.

It should also be understood that all the aspects identified as preferred and advantageous for the composition and its components are to be considered similarly preferred and advantageous also for the preparation and uses of the composition itself.

Below are working examples of the present invention provided for illustrative and nonlimiting purposes.

### EXAMPLES

### Example 1.

The following solution, according to the present invention, has been prepared:

| | % by weight |
|---|---|
| N-acetylcysteine | 1.20 |
| tris(hydroxymethyl) aminomethane | 3.60 |
| disodium EDTA | 1.00 |
| water | balance to 100.00 |

### Example 2.

The following solution, according to the present invention, has been prepared:

| | % by weight |
|---|---|
| N-acetylcysteine | 2.40 |
| tris(hydroxymethyl) aminomethane | 7.20 |
| disodium EDTA | 2.00 |
| water | balance to 100.00 |

### Example 3. (reference example)

The following solution, according to the present invention, has been prepared:

| | % by weight |
|---|---|
| N-acetylcysteine | 2.40 |
| tris(hydroxymethyl) aminomethane | 1.80 |
| disodium EDTA | 0.50 |
| water | balance to 100.00 |

### Example 4.

The following solution, according to the present invention, has been prepared:

| | % by weight |
|---|---|
| N-acetylcysteine | 1.20 |
| tris(hydroxymethyl) aminomethane | 3.60 |
| disodium EDTA | 1.00 |
| propylenic glycol | 2.00 |
| water | balance to 100.00 |

### Example 5.

The following solution, according to the present invention, has been prepared:

| | % by weight |
|---|---|
| N-acetylcysteine | 2.40 |
| tris(hydroxymethyl) aminomethane | 7.20 |
| disodium EDTA | 2.00 |
| propylenic glycol | 4.00 |
| water | balance to 100.00 |

### Example 6.

### Stability test of the compositions of the Examples 1-5

The compositions prepared above were placed in an oven at 60°C for 1 month in order to verify the overall stability of the same and to evaluate the possible release of odorous compounds, under conditions of storage accelerated by thermal stress.

At the end of the test month, all the compositions were perfectly odourless, as well as transparent as freshly prepared.

The efficacy of the stabilizing agent was confirmed in its stabilizing action of N-acetylcysteine, such as to contrast, surprisingly even under particularly strong conditions, the degradation thereof. This makes possible the use of N-acetylcysteine also in external topical applications, such as the prevention and treatment of otitis, since the obstacles of unpleasantness of use and instability over time are no longer present.

## Claims

1. A pharmaceutical composition comprising N-acetylcysteine and a stabilizing agent, wherein
said stabilizing agent comprises
a buffer compound is selected from TRIS (or tris(hydroxymethyl) aminomethane), PIPES (or piperazin-1,4-bis(2-ethanesulfonate acid)), TRIS*HCl, HEPES (or 4-2-hydroxyethyl-1-piperazinyl-ethanesulfonic acid), monobasic and dibasic sodium phosphate, or citric acid, and
a sequestering compound selected from EGTA (ethyleneglycoltetraacetic acid), EDTA (ethylenediaminetetraacetic acid) or its salified anhydrous or hydrated form, calcium-disodium EDTA or its hydrated form, diammonium EDTA or its hydrated form, bipotassium EDTA or its hydrated form, bisodium EDTA or its hydrated or dihydrated form, TEA-EDTA (EDTA mono salt (triethanolamine)), tetrasodium EDTA, tripotassium EDTA, trisodium EDTA, HEDTA (hydroxyethyl-ethylenediaminotriacetic acid), HEDTA-EDTA, and mixtures thereof,
said stabilizing agent is in a weight quantity higher than N-acetylcysteine, and
said composition has a pH of 7-9 in water.

2. The composition of claim 1, wherein stabilizing agent and N-acetylcysteine are in a weight ratio of at least 1.2:1.

3. The composition of claim 1 or 2, wherein stabilizing agent and N-acetylcysteine are in a weight ratio of 1.5: 1 to 20: 1, preferably 1.5:1 to 10:1.

4. The composition of any one of claims 1-3, wherein, in the stabilizing agent, said buffer compound is in a quantity higher than said sequestering compound.

5. The composition of claim 4, wherein said buffer compound and said sequestering compound are in a weight ratio of 1.1:1 to 20:1, preferably 1.5:1 to 10:1.

6. The composition of any one of claims 1-5, comprising up to 10 wt% of stabilizing agent, based on the weight of the composition.

7. The composition of claim 6, comprising up to 8 wt% of stabilizing agent, based on the weight of the composition, preferably comprising 0.1-5 wt% of stabilizing agent, based on the weight of the composition.

8. The composition of any one of claims 1-7, comprising N-acetylcysteine, TRIS, disodium EDTA, and water.

9. The composition of any one of claims 1-8, comprising up to 5 wt% of N-acetylcysteine, based on the weight of the composition.

10. The composition of any one of claims 1 to 9 for use in the prevention and treatment of cutaneous affections and external otitis and otitis media, preferably in pets.

## Patentansprüche

1. Pharmazeutische Zusammensetzung, umfassend N-Acetylcystein und ein Stabilisierungsmittel, wobei
das Stabilisierungsmittel Folgendes umfasst
eine Pufferverbindung, ausgewählt aus TRIS (oder Tris(hydroxymethyl)aminomethan), PIPES (oder Piperazin-1,4-bis(2-ethansulfonatsäure)), TRIS^{∗}HC1, HEPES (oder 4-2-Hydroxyethyl-l-piperazinyl-ethansulfonsäure), einbasigem und zweibasigem Natriumphosphat oder Zitronensäure, und
eine sequestrierende Verbindung, ausgewählt aus EGTA (Ethylenglykoltetraessigsäure), EDTA (Ethylendiamintetraessigsäure) oder ihrer wasserfreien oder hydratisierten Salzform, Calcium-Dinatrium-EDTA oder ihrer hydratisierten Form, Diammonium-EDTA oder ihrer hydratisierten Form, Bikalium-EDTA oder ihre hydratisierte Form, Bisodium-EDTA oder ihre hydratisierte oder dihydratisierte Form, TEA-EDTA (EDTA-Monosalz (Triethanolamin)), Tetranatrium-EDTA, Trikalium-EDTA, Trinatrium-EDTA, HEDTA (Hydroxyethyl-Ethylendiamintriessigsäure), HEDTA-EDTA und deren Gemische,
wobei das Stabilisierungsmittel in einer höheren Gewichtsmenge als N-Acetylcystein ist, und die Zusammensetzung in Wasser einen pH von 7-9 aufweist.

2. Zusammensetzung nach Anspruch 1, wobei Stabilisierungsmittel und N-Acetylcystein in einem Gewichtsverhältnis von mindestens 1,2:1 sind.

3. Zusammensetzung nach Anspruch 1 oder 2, wobei Stabilisierungsmittel und N-Acetylcystein in einem Gewichtsverhältnis von 1,5:1 bis 20:1, vorzugsweise 1,5:1 bis 10:1, sind.

4. Zusammensetzung nach einem der Ansprüche 1 bis 3, wobei in dem Stabilisierungsmittel die Pufferverbindung in einer größeren Menge ist als die sequestrierende Verbindung.

5. Zusammensetzung nach Anspruch 4, wobei die Pufferverbindung und die sequestrierende Verbindung in einem Gewichtsverhältnis von 1,1:1 bis 20:1, vorzugsweise 1,5:1 bis 10:1, sind.

6. Zusammensetzung nach einem der Ansprüche 1 bis 5, umfassend bis zu 10 Gewichtsprozent Stabilisierungsmittel, bezogen auf das Gewicht der Zusammensetzung.

7. Zusammensetzung nach Anspruch 6, umfassend bis zu 8 Gewichtsprozent Stabilisierungsmittel, bezogen auf das Gewicht der Zusammensetzung, vorzugsweise umfassend 0,1-5 Gewichtsprozent Stabilisierungsmittel, bezogen auf das Gewicht der Zusammensetzung.

8. Zusammensetzung nach einem der Ansprüche 1-7, umfassend N-Acetylcystein, TRIS, Dinatrium-EDTA und Wasser.

9. Zusammensetzung nach einem der Ansprüche 1-8, umfassend bis zu 5 Gewichtsprozent N-Acetylcystein, bezogen auf das Gewicht der Zusammensetzung.

10. Zusammensetzung nach einem der Ansprüche 1 bis 9 zur Verwendung bei der Vorbeugung und Behandlung von Hautkrankheiten und äußerer Otitis und Otitis media, vorzugsweise bei Haustieren.

## Revendications

1. Composition pharmaceutique comprenant de la N-acétylcystéine et un agent stabilisant, ledit agent stabilisant comprenant
un composé tampon choisi parmi le TRIS (ou tris(hydroxyméthyl)aminométhane), le PIPES (ou pipérazine-1,4-bis(acide 2-éthanesulfate)), le TRIS^{∗}HCl, le HEPES (ou 4-2-hydroxyéthyl-1-pipérazinyl-acide éthanesulfonique), le phosphate de sodium monobasique et dibasique, ou l'acide citrique, et
un composé séquestrant choisi parmi l'EGTA (acide éthylène glycol tétraacétique), l'EDTA (acide éthylène diamine tétraacétique) ou sa forme salifiée anhydre ou hydratée, l'EDTA calcio-disodique ou sa forme hydratée, l'EDTA diammonique ou sa forme hydratée, l'EDTA bipotassique ou sa forme hydratée, l'EDTA bisodique ou sa forme hydratée ou dihydratée, le TEA-EDTA (mono sel d'EDTA (triéthanolamine)), l'EDTA tétrasodique, l'EDTA tripotassique, l'EDTA trisodique, le HEDTA (acide hydroxy éthyléthylène diamino triacétique), le HEDTA-EDTA, et leurs mélanges,
ledit agent stabilisant se trouvant en une quantité pondérale supérieure à la N-acétylcystéine, et ladite composition comportant un pH de 7 à 9 dans l'eau.

2. Composition selon la revendication 1, ledit agent stabilisant et ladite N-acétylcystéine se trouvant dans un rapport pondéral d'au moins 1,2:1.

3. Composition selon la revendication 1 ou 2, ledit agent stabilisant et ladite N-acétylcystéine se trouvant dans un rapport pondéral de 1,5:1 à 20:1, de préférence de 1,5:1 à 10:1.

4. Composition selon l'une quelconque des revendications 1 à 3, dans ledit agent stabilisant, ledit composé tampon se trouvant en quantité supérieure par rapport audit composé séquestrant.

5. Composition selon la revendication 4, ledit composé tampon et ledit composé séquestrant se trouvant dans un rapport pondéral de 1,1:1 à 20:1, de préférence de 1,5:1 à 10:1.

6. Composition selon l'une quelconque des revendications 1 à 5, comprenant jusqu'à 10 % en poids d'agent stabilisant, par rapport au poids de la composition.

7. Composition selon la revendication 6, comprenant jusqu'à 8 % en poids d'agent stabilisant, par rapport au poids de la composition, comprenant de préférence 0,1 à 5 % en poids d'agent stabilisant, par rapport au poids de la composition.

8. Composition selon l'une quelconque des revendications 1 à 7, comprenant de la N-acétylcystéine, du TRIS, de l'EDTA disodique et de l'eau.

9. Composition selon l'une quelconque des revendications 1 à 8, comprenant jusqu'à 5 % en poids de N-acétylcystéine, par rapport au poids de la composition.

10. Composition selon l'une quelconque des revendications 1 à 9, destinée à être utilisée dans la prévention et le traitement des affections cutanées et d'une otite externe et d'une otite moyenne, de préférence chez les animaux de compagnie.
